# EUROPEAN PATENT APPLICATION

(11) **EP 2 433 636 A1**
(43) Date of publication of application: **28.03.2012**
(21) Application number: 10180033.2
(22) Date of filing: 27.09.2010
(51) Int. Cl.: A61K 31/506, A61P 35/00

(54) **Treatment of Malignant Diseases**

(71) Applicant: Medizinische Universität Wien, 1090 Wien (AT)
(72) Inventor: The designation of the inventor has not yet been filed
(74) Representative: Sonn & Partner Patentanwälte

(57) **Abstract**

The invention discloses a compound selected from the group N*2*,N*4*-Bis-(2-methyl-1H-indol-5-yl)-pyrimidine-2,4-diamine, N*2*-(4-Diethylamino-1-methyl-butyl)-5-methyl-N*4*-(2-methyl-1H-indol-5-yl)-pyrimidine-2,4-diamine, N*2*,N*4*-Bis-(1H-indol-5-yl)-pyrimidine-2,4-diamine and N*2*-(1H-Indol-6-yl)-N*4*-(1H-indol-5-ylmethyl)-pyrimidine-2,4-diamine, preferably N*2*,N*4*-Bis-(2-methyl-1H-indol-5-yl)-pyrimidine-2,4-diamine,
and any of its stereoisomeric forms or a mixture thereof;
or a salt or solvate of a compound of these compounds, especially for use in the treatment of malignant diseases.

## Description

The present invention relates to the use of inhibitors of Rac GTPases for the treatment of malignant diseases.

Rho family GTPases are important intracellular signaling proteins that control diverse cellular functions related to cancer development, including actin cytoskeleton organization, transcription regulation, cell cycle progression, apoptosis, vesicle trafficking, and cell-to-cell and cell-to-extracellular matrix adhesions. They have emerged as potentially useful targets for tumourigenesis. In particular, several lines of evidence indicate that the Rho family member, Rac, may play critical roles in several aspects of tumourigenesis and cancer progression. First, constitutively active Rac1 could accelerate cell growth and transform NIH 3T3 cells, whereas dominant negative Rac inhibited cell growth. Second, Rac1 was found to be an important component of Ras-induced transformation, and the Rac-specific GEF (Guanine nucleotide-exchange factor), Tiam1, is involved in Ras-mediated skin cancer progression in mice. Third, loss of function in PTEN, p19Arf, or p53 tumour suppressor leads to elevation of Rac activity, resulting in increased migration and proliferation in PTEN^{-/-}, p19Arf^{-/-}, or p53^{-/-} cells. Fourth, hyperactive Rac1 and Rac3 were associated with the increased proliferation of several breast cancer cell lines. In addition, a point mutation of Tiam1 has been identified in human tumours, and this mutant was shown to transform NIH 3T3 cells. Unlike Ras, however, constitutively active mutation of Rac has not been found in human cancer. It is possible that up-regulation or overexpression, rather than GTPase-defective mutation, of Rac is associated with tumourigenic properties. Therefore, the signaling step of Rac1 activation by GEF could be a targeting site of the signaling chains involving Rac. Based on the structural information provided by the Rac1-Tiam1 complex and by taking the computer-simulated virtual screening approach, the small chemical compound of the National Cancer Institute chemical database, NSC23766 (N6-(2-((4-(diethylamino)-1- methylbutyl) amino)-6-methyl-4-pyrimidinyl)-2-methyl-4, 6-quinolinediamine), was identified as a Rac-specific inhibitor (Gao et al., PNAS 101 (2004), 7618-7623; WO 2005/051392 A1; US 2006/0004032 A1). It was shown that this compound was able to discriminate Rac1 from Cdc42 and RhoA and specifically inhibited Rac1 activation by its GEF in vitro and in vivo. This compound was able to inhibit proliferation, anchorage independent growth, and invasion of the Rac-hyperactive prostate cancer cells (in a human cancer cell line, PC-3) by down-regulating the endogenous Rac1 activity. As a first generation inhibitor for Rac, NSC23766 is capable of specifically interfering with Rac1 but not with Cdc42 or RhoA activation by their respective GEFs, as demonstrated by the in vitro binding and exchange assays and the in vivo Rho GTPase effector domain pull-down assays.

Echeverria et al. (Arch. Pharm. Chem. Life Sci., 339 (2006):182-192) disclose apoptosis inducers and Caspase-3 Activators. Beaupre et al. (Invest. New Drugs, 17 (1999):137-143) disclose biologic considerations and clinical applications of RAS inhibitors in hematologic cancers. WO 03/102139 A2 discloses erbin as Ras-inhibitors.

It is an object of the present invention to provide further substances treating tumours identified by their Rac inhibiting property. These substances should be small molecules with molecular weights not exceeding 1000 (or even 500) Da.

Therefore, the present invention relates to a compound selected from N*2*-(4-Diethylamino-1-methyl-butyl)-5-methyl-N*4*-(2-methyl-1H-indol-5-yl)-pyrimidine-2,4-diamine, N*2*,N*4*-Bis-(1H-indol-5-yl)-pyrimidine-2,4-diamine, N*2*,N*4*-Bis-(2-methyl-1H-indol-5-yl)-pyrimidine-2,4-diamine or N*2*-(1H-Indol-6-yl)-N*4*-(1H-indol-5-ylmethyl)-pyrimidine-2,4-diamine, preferably N*2*,N*4*-Bis-(2-methyl-1H-indol-5-yl)-pyrimidine-2,4-diamine, or any of its stereoisomeric forms or a mixture thereof; or a salt or solvate of these compounds, especially for use in the treatment of malignant diseases.

These compounds N*2*-(4-Diethylamino-1-methyl-butyl)-5-methyl-N*4*-(2-methyl-1H-indol-5-yl)-pyrimidine-2,4-diamine, N*2*,N*4*-Bis-(1H-indol-5-yl)-pyrimidine-2,4-diamine, N*2*,N*4*-Bis-(2-methyl-1H-indol-5-yl)-pyrimidine-2,4-diamine and N*2*-(1H-Indol-6-yl)-N*4*-(1H-indol-5-ylmethyl)-pyrimidine-2,4-diamine which are hereinafter referred to as "the compounds according to the present invention" (especially N*2*,N*4*-Bis-(2-methyl-1H-indol-5-yl)-pyrimidine-2,4-diamine, hereinafter referred to as "the preferred compound according to the present invention") have been identified in the course of the present invention as being potent and specific Rac1 inhibitors. The compounds according to the present invention have been identified as preferred agents for cancer treatment in a diligently performed screening and selection process based on specialised and specific in vitro and in vivo studies.

The compounds according to the present invention are efficient in a broad range of malignancies, since the Rac system plays a role in solid malignant tumours as well as in hematopoietic malignancies. Accordingly, the compounds of the present invention may preferably be used for the treatment of carcinomas (including breast, colon, rectum, esophagus, stomach, liver, lung, renal (especially renal cell tumour and adrenal tumour), thyroid, ovary, uterus, vagina, pancreas, bladder, prostate, skin, and head and neck squamous cell carcinoma (HNSCC), as well as oral squamous cell carcinoma (OSCC)), hematopoietic malignancies of lymphoid lineage (including anaplastic large cell lymphomas (ALCLs), p210-BCR-ABL-dependent leukemia and hairy cell leukemia), hematopoietic malignancies of myeloid lineage (including chronic myelogenous leukemias (CML)), malignant tumours of mesenchymal origin (including rhabdomyosarcoma), and malignant tumours of neuronal origin, especially glioblastoma.

The route of administration can be chosen and optimised by the physician based on the tumour type, the physical status of the patient (and the tumour stage) and the respective compound. The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels or aqueous or oily solutions or suspensions) for administration by inhalation (for example as a finely divided powder or a liquid aerosol), for administration by insufflation (for example as a finely divided powder) or for parenteral administration (for example as a sterile aqueous for intravenous or sterile or oily solution for subcutaneous, intramuscular application) or as a suppository for rectal application. Of course, besides administration via injection (syringes or infusion techniques) also other administration techniques may be applied. Intratumoural injections can be used for treatment of cancers as well.

One embodiment provides for a method for reducing cancer cell proliferation by administering in a subject having cancer an effective amount of a compound according to the present invention.

Another embodiment provides for the use of an effective amount of an active compound according to the present invention for the preparation of pharmaceutical composition for the treatment of a disease associated with abnormal cell proliferation.

Because of their cell proliferation inhibitory activity, the compounds according to the present invention are suitable for treating a variety of malignant diseases in a variety of conditions. Accordingly, the compounds can be used at very early stages of a disease, or after significant progression, including metastasis. The term "treatment" or "treating" designates in particular a reduction of the burden in a patient (whom is hereinafter always understood as human patient, except explicitly indicated otherwise; this holds also true for the using of the words "body" or "cell", which - unless explicitly stated otherwise - refer to human cells or the human body), such as a reduction in cell proliferation rate, a destruction of diseased proliferative cells, a reduction of tumour mass or tumour size, a delaying of tumour progression, as well as a complete tumour suppression.

The compounds of the preferred embodiments are particularly suited for the treatment of cancers, such as solid malignant tumours or malignancies of the hematopoietic system. Specific examples include leukemia, prostate cancer, ovarian cancer, uterine cancer, vaginal cancer, pancreas cancer, lung cancer, breast cancer, liver cancer, head and neck cancer, colon cancer, rectal cancer, esophagus cancer, stomach cancer, skin cancer, bladder cancer and non-Hodgkin's lymphoma cancer. The most preferred embodiments include the treatment of prostate cancer.

The active compounds of the present invention can be administered through various routes, typically by injection, for local or systemic effect(s). Intravenous administration of the compounds according to the present invention being the most preferred route of administration. Intratumoural injections can also be used for treating existing cancers. However, besides the preferred intravenous administration, other administration routes can be used as well, such as intramuscular, intradermic, subcutaneous, etc. Furthermore, repeated injections can be performed, if needed, although it is believed that limited injections will be needed in view of the efficacy of the compounds.

It is contemplated that such target cells can be located within an animal or a patient, in which case a safe and effective amount of the complex, in pharmacologically acceptable form, would be administered. Generally speaking, it is contemplated that useful pharmaceutical compositions of the preferred embodiments will include the selected active compound derivative in a convenient amount, e. g., from about 0.001% to about 10% (w/w) that is diluted in a pharmacologically or physiologically acceptable carrier, such as, for example, phosphate buffered saline. The route of administration and ultimate amount of material that is administered to the patient or animal under such circumstances will depend upon the intended application and will be apparent to those of skill in the art in light of the examples which follow.

Any composition chosen should be of low or non-toxicity to the cell or to the body in general. Toxicity for any given compound can vary with the concentration of compound used. It is also beneficial if the compound chosen is metabolized or eliminated by the body and if this metabolism or elimination is done in a manner that will not be harmfully toxic. In any way, the compounds of the present invention are preferably administered such that a therapeutically effective concentration of the compound is in contact with the affected cells of the body.

The dose administered, particularly to a patient, in the context of the preferred embodiments is preferably sufficient to effect a therapeutic response in the patient over a reasonable period of time. The dose will be determined by the strength of the particular compound employed and the condition of the patient, as well as the body weight of the patient to be treated. The existence, nature, and extent of any adverse side effects that might accompany the administration of a particular compound also will determine the dose and the particular route of administration employed with a particular patient and a particular cancer type. In general, the compounds of the preferred embodiments are therapeutically effective at low doses. The generally preferred dose range is from about 0.0001 to about 10 mM. Preferably, the effective dose range is from about 0.001, 0.005, 0.01, 0.05, 0.06, 0.07, 0.08, or 0.09 mM, to about 0.1, 0.2, 0.3, 0.4, 0.5, 0.6, 0.7, 0.8, 0.9, or 1 mM.

The compound can be administered in a pharmaceutically acceptable carrier. Pharmaceutically acceptable carriers are well-known to those who are skilled in the art. The choice of carrier will be determined in part by the particular compound, as well as by the particular method used to administer the composition. Accordingly, there is a wide variety of suitable formulations of the pharmaceutical composition of the preferred embodiments.

The compounds can be administered orally, topically, parenterally, by inhalation (e.g. with a spray), vaginally, rectally or sublingually in dosage unit formulations. The term "administration by injection" includes but is not limited to parenteral injections, such as intravenous, intraarticular, intramuscular and subcutaneous injections, if desired also by use of infusion techniques. For example, osmotic minipumps or different types of depots may be applied which can be implanted to deliver a daily, two-day, weekly, bi-weekly, etc. dose in a controlled administration can include topical application which can enter e.g. the blood system by transdermal passage. One or more compounds can be present in association with one or more non-toxic pharmaceutically acceptable carriers and if desired other active ingredients.

Compositions intended for oral use can be prepared according to any suitable method known to the art for the manufacture of pharmaceutical compositions.

Such compositions can contain one or more agents selected from the group consisting of diluents, sweetening agents, flavouring agents, colouring agents and preserving agents in order to provide palatable preparations. Tablets contain the active ingredient in admixture with non-toxic pharmaceutically acceptable excipients that are suitable for the manufacture of tablets. These excipients can be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets can be uncoated or they can be coated by known techniques to sustain absorption in the gastrointestinal tract and thereby provide a sustained action over a longer period. For example, a time delay material such as glyceryl monostearate or glyceryl distearate can be employed. These compounds can also be prepared in solid, rapidly released form.

Formulations for oral use can also be presented as hard capsules wherein the active ingredient is mixed with an inert solid diluent, for example, calcium carbonate, calcium phosphate or kaolin, or as soft capsules wherein the active ingredient may be mixed with an oil medium, for example peanut oil, liquid paraffin or olive oil. Capsules can e.g. be made of gelatin or other substances known to serve this purpose.

Aqueous suspensions containing the active materials in admixture with excipients suitable for the manufacture of aqueous suspensions can also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavouring and colouring agents, can also be present.

The compounds according to the present invention can also be in the form of non-aqueous liquid formulations, e. g., oily suspensions which can be formulated by suspending the active ingredients in a vegetable oil, for example arachis oil, olive oil, sesame oil or peanut oil, or in a mineral oil such as liquid paraffin. The oily suspensions can contain a thickening agent, for example beeswax, hard paraffin or cetyl alcohol. Sweetening agents such as those set forth above, and flavouring agents can be added to provide palatable oral preparations. These compositions can be preserved by the addition of an anti-oxidant.

Compounds according to the present invention can also be administrated topically with systemic effect (by transdermal delivery) using methods known to those skilled in the art. For example, a solution or suspension of an active agent in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of an active agent can be formulated into a lotion or salve.

Suitable solvents for processing transdermal delivery systems are known to those skilled in the art, and include lower alcohols such as ethanol or isopropyl alcohol, lower ketones, lower carboxylic acid esters such as ethyl acetate, polar ethers such as tetrahydrofuran, lower hydrocarbons such as hexane or cyclohexane, or halogenated hydrocarbons such as dichloromethane, trichlorotrifluoroethane, or trichlorofluoroethane. Suitable solvents can also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to about 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations can also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates can also be used as matrix components. Additional additives, such as viscous resins or oils can be added to optimise the viscosity of the matrix.

Pharmaceutical compositions according to the present invention can also be in the form of oil-in-water emulsions. The oil phase can be a vegetable oil, for example olive oil or arachis oil, or a mineral oil, for example, liquid paraffin or mixtures of these. Suitable emulsifying agents can be naturally-occurring gums, for example, gum acacia or gum tragacanth, naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions can also contain sweetening and flavouring agents. Syrups and elixirs can be formulated with sweetening agents, for example glycerol, propylene glycol, sorbitol or sucrose. Such formulations can also contain a demulcent, a preservative and flavouring and colouring agents.

The compounds of the present invention can also be administered in the form of suppositories for rectal or vaginal administration of the drug. These compositions can be prepared by mixing the drug with a suitable nonirritating excipient which is solid at ordinary temperatures but liquid at the rectal temperature or vaginal temperature and will therefore melt in the rectum or vagina to release the drug. Such materials include hard, such as Witepsol^{®} butter and polyethylene glycols.

For all regimens of use disclosed herein for the active agent, the daily dosage regimen will preferably be from about 0.0001 mg to about 100 mg, especially from 0.005 mg to 50 mg/Kg of total body weight. Preferably, the daily oral dosage regimen will be from about 0.001 mg to about 20 mg/Kg of total body weight. The daily dosage for administration by injection, including parenteral injections, such as intravenous, intramuscular, and subcutaneous injections, and use of infusion techniques will preferably be from 0.0001 to 2 mg/Kg of total body weight.

Preferably, the daily dosage for administration by injection, including parenteral injections, such as intravenous, intramuscular, and subcutaneous injections, and use of infusion techniques will preferably be from about 0.0001, 0.0005, 0.001, 0.005, 0.01, 0.02, 0.03, 0.04, or 0.05 to about 0.1, 0.5, 1, 1.1, 1.2, 1.3, 1.4, 1.5, 1.6, 1.7, 1.8, 1.9, 2, or 4 mg/Kg of total body weight. The daily vaginal dosage regime will preferably be from 0.001 to 20 mg/Kg of total body weight. The daily topical dosage regimen will preferably be from 0.01 to 40 mg /Kg administered between one to four times daily. The concentration for vaginal dosage and topical dosage will preferably be that required to maintain a daily dosage from 0.01 to 40 mg/Kg. Preferably, the daily oral dosage regimen will be from about 0.001, 0.005, 0.01, 0.05, 0.1, 0.2, 0.3, 0.4, or 0.5 to about 1, 5, 10, 11, 12, 13, 14, 15, 16, 17, 18, 19, or 20 mg/Kg of total body weight. The daily inhalation dosage regimen will preferably be from 0.001 to 20 mg/Kg of total body weight. Preferably, the daily inhalation dosage regimen will be from about 0.001, 0.005, 0.01, 0.05, 0.1, 0.2, 0.3, 0.4, or 0.5 to about 1, 5, 10, 11, 12, 13, 14, 15, 16, 17, 18, 19, or 20 mg/Kg of total body weight.

It will be appreciated by those skilled in the art that the particular method of administration will depend on a variety of factors, all of which are considered routinely when administering therapeutics, especially tumour therapeutics. It will also be understood, however, that the specific dose level for any given patient will depend upon a variety of factors, including, the activity of the specific compound employed, the age of the patient, the body weight of the patient, the general physical condition of the patient, the gender of the patient, the diet of the patient, time of administration, route of administration, rate of excretion, drug combinations, and the severity and stage of the disease undergoing therapy. It will be further appreciated by one skilled in the art that the optimal course of treatment, i. e., the mode of treatment and the daily number of doses of an active agent or a pharmaceutically acceptable salt thereof given for a defined number of days, can be ascertained by those skilled in the art using conventional treatment tests.

The modulation of oncogenic transforming activity by an active agent according to the present invention can be applied in practice by various procedures.

For example, the present invention relates to a method of treating cancer comprising administering, to a subject in need of treatment, an amount of a compound according to the present invention effective to treat the disease. Treating the disease can mean delaying its onset, delaying the progression of the disease, improving or delaying clinical and pathological signs of disease.

The present invention provides an improved method for treatment of malignant diseases comprising administration of a pharmaceutically effective quantity of the compounds according to the present invention or its pharmaceutically acceptable salts or esters, active agent analogs or their pharmaceutically acceptable salts or esters, or a combination thereof.

The compositions and preparations described preferably contain at least 0.01% (weight percentage), preferably at least 0.1%, of a compound according to the present invention. The percentage of the compositions and preparations can, of course, be varied, and can contain between about 0.2% and 60%, preferably between 0.3% to 10%, especially 0.5% to 5%, of the weight of the amount administered. The amount of active compounds in such pharmaceutically useful compositions and preparations is such that a suitable dosage will be obtained.

The compounds according to the present invention may also be administered in the form of salts. Accordingly, any reference to a compound of the present invention as used herein is understood to include reference to salts thereof, unless otherwise indicated. The term "salt(s)", as employed herein, denotes acidic and/or basic salts formed with inorganic and/or organic acids and bases. In addition, since the active agent according to the present invention contains a pyridine and indol ring as a basic moiety, salts involving these basic moieties in salt formation are specifically preferred. Pharmaceutically acceptable (i. e., non-toxic, physiologically acceptable) salts are preferred, although other salts are also useful, e. g., in isolation or purification steps, which can be employed during preparation. Salts of the compounds of the active agent can be formed, for example, by reacting a compound of the present invention with an amount of acid or base, such as an equivalent amount, in a medium such as one in which the salt precipitates or in an aqueous medium followed by lyophilization.

Since the active agent according to the present invention contains basic moieties, such as the pyridine or indol ring, salts can easily be formed with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3- phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Prodrugs and solvates of the compounds of the present invention are also contemplated herein. The term "prodrug", as employed herein, denotes a compound which, upon administration to a subject, undergoes chemical conversion by metabolic or chemical processes to yield a compound of the present invention, and/or a salt and/or solvate thereof. Solvates of the active agent are preferably hydrates.

All stereoisomers of the present compounds, such as those, for example, which can exist due to asymmetric carbons on any of the substituents, including enantiomeric forms (which can exist even in the absence of asymmetric carbons) and diastereomeric forms, are contemplated and within the scope of the preferred embodiments. Individual stereoisomers of the compounds of the preferred embodiments can, for example, be substantially free of other isomers, or can be admixed, for example, as racemates or with all other or other selected stereoisomers. The chiral centers of the preferred embodiments can have the S or R configuration as defined by the IUPAC 1974 Recommendations.

When the compounds according to the present invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts.

Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

Mixture of solvents can be used. Organic bases like lysine, arginine, diethanolamine, choline, guandine and their derivatives etc. can also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, sulfonic acid, acetic acid, citric acid, maleic acid, salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane, etc. Mixtures of solvents can also be used.

The compounds according to the present invention can be formulated in various forms, including solid and liquid forms, such as tablet, gel, syrup, powder, aerosol, etc.

The compositions to be administered according to the present invention can contain physiologically acceptable excipients, such as diluents, fillers, lubricants, solvents, binders, stabilizers, and the like. Diluents that can be used in the compositions include but are not limited to dicalcium phosphate, calcium sulphate, lactose, cellulose, kaolin, mannitol, sodium chloride, dry starch, powdered sugar and - for prolonged release tablets -hydroxy propyl methyl cellulose (HPMC). The binders that can be used in the compositions include but are not limited to starch, gelatin and fillers such as sucrose, glucose, dextrose and lactose.

Natural and synthetic gums that can be used in the compositions include but are not limited to sodium alginate, Ghatti gum, carboxymethyl cellulose, methyl cellulose, polyvinyl pyrrolidone and veegum. Excipients that can be used in the compositions include but are not limited to microcrystalline cellulose, calcium sulfate, calcium phosphate, starch, magnesium stearate, lactose, and sucrose. Stabilizers that can be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.

Solvents that can be used include but are not limited to Ringers solution, water, distilled water, dimethyl sulfoxide to 50% in water, propylene glycol (neat or in water), phosphate buffered saline, balanced salt solution, glycol and other conventional fluids.

The compounds of the present invention can be used in a substantially similar manner to other known anti-tumour agents for treating (both chemopreventively and therapeutically) various malignant diseases. For the compounds of the present invention, the anti-tumour dose to be administered, whether a single dose, multiple doses, or a daily dose, will of course vary with the particular compound employed because of the varying potency of the compound, the chosen route of administration, the body weight and body surface of the recipient, the type of tumour, and the patient's physical condition and disease stage. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. A skilled physician in the art of cancer treatment will be able to ascertain, without undue experimentation, appropriate protocols for the effective administration of the compounds of the preferred embodiments, such as by referring to the earlier published studies on compounds found to have anti-tumour properties.

The present invention is further disclosed by the following examples and the drawing figures, yet without being restricted thereto.

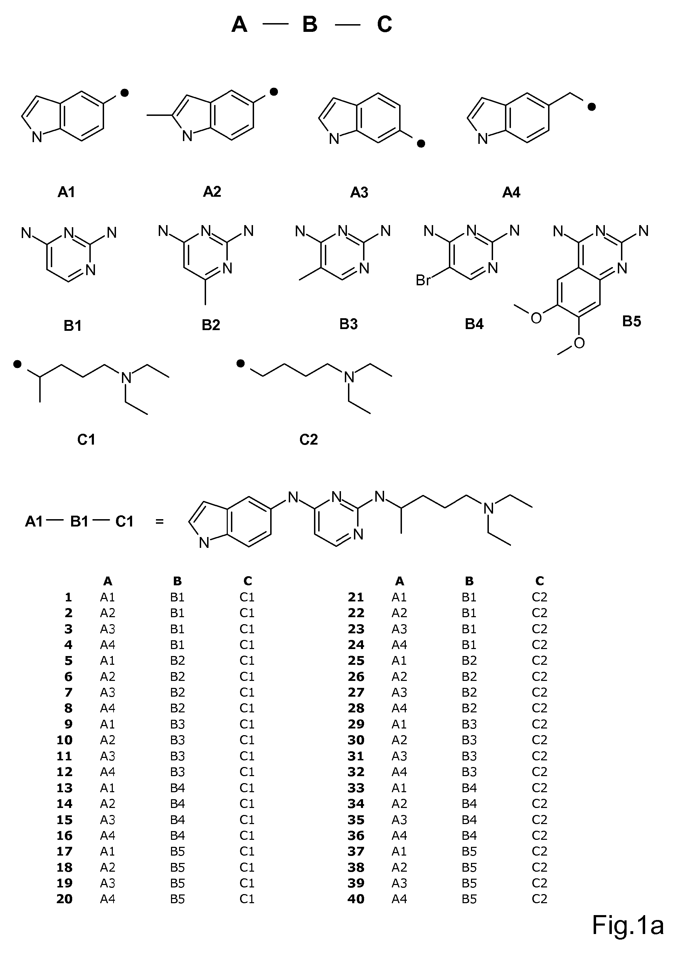

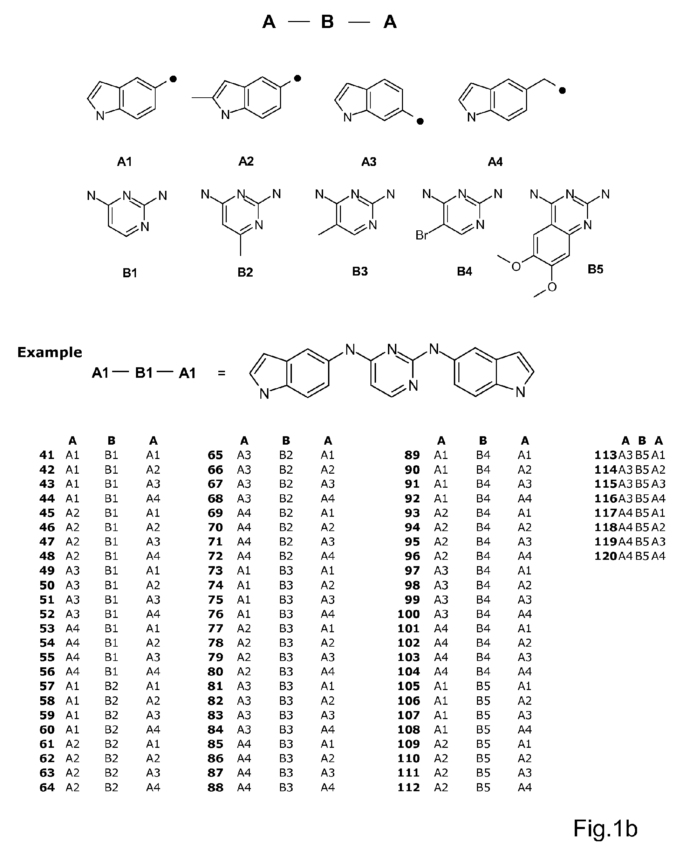

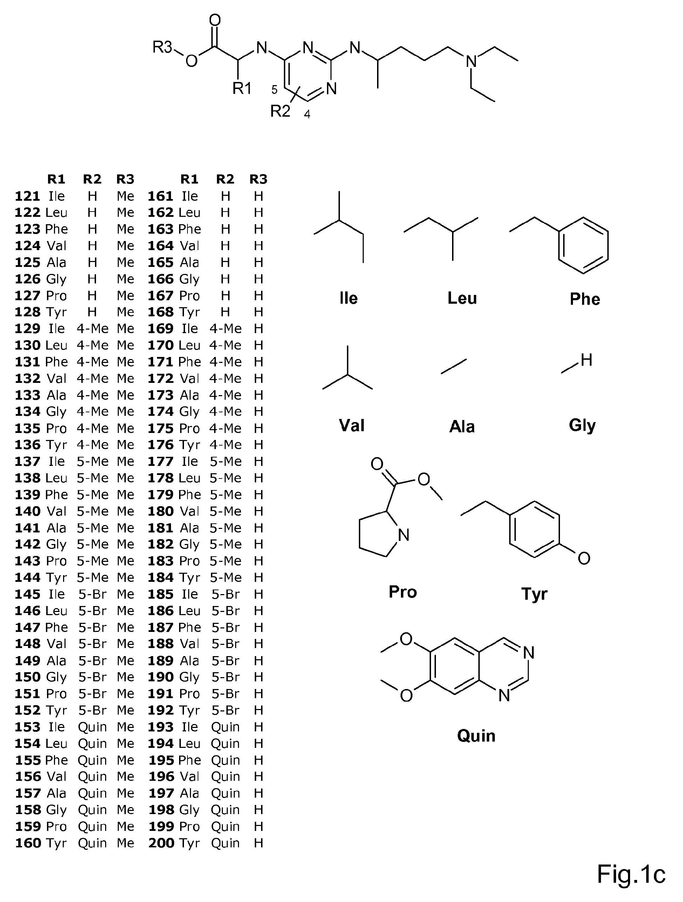

Fig. 1a, b, c show the chemical formula of the compounds screened in the course of the present invention (in the formulae, H atoms are not depicted);

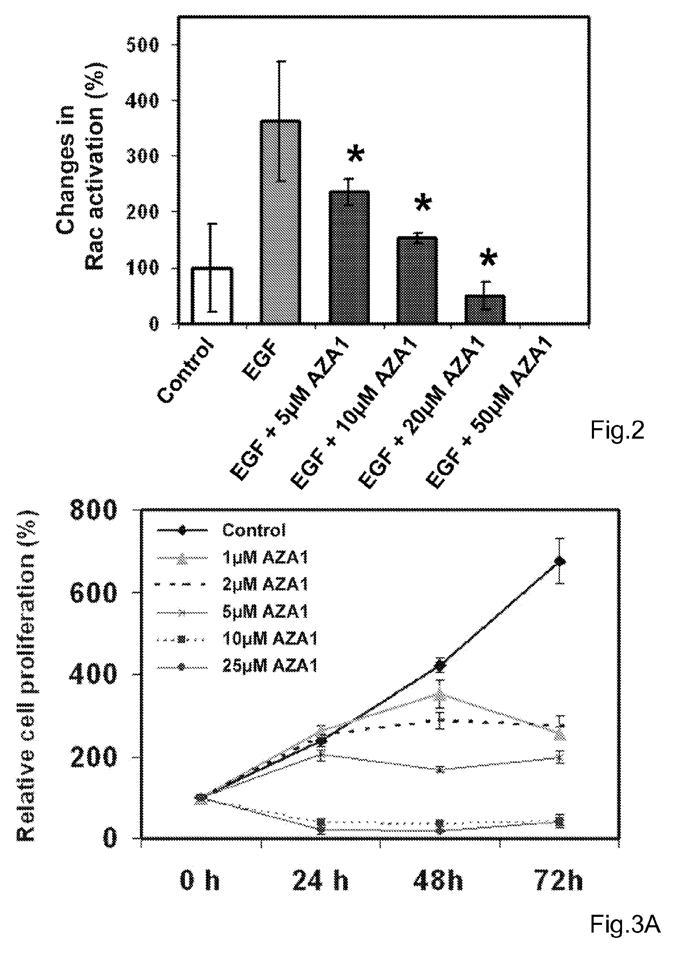

Fig. 2 shows that Compound AZA1 inhibits Rac activation. Rac activation in CRL-2505 prostate cancer cells was tested before and after 4h of incubation with different concentrations of compound AZA1 and stimulation with 50 ng/ml EGF by a Rac 1, 2, 3 activation assay. Data shown represent one representative experiment of three experiments. *, P<0.05, significantly different from EGF.

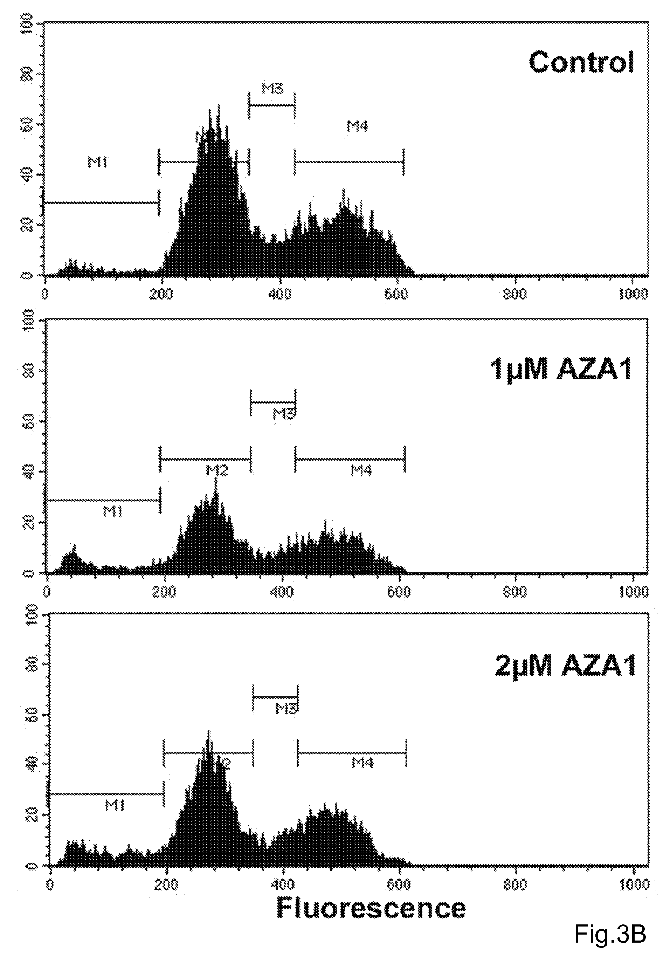

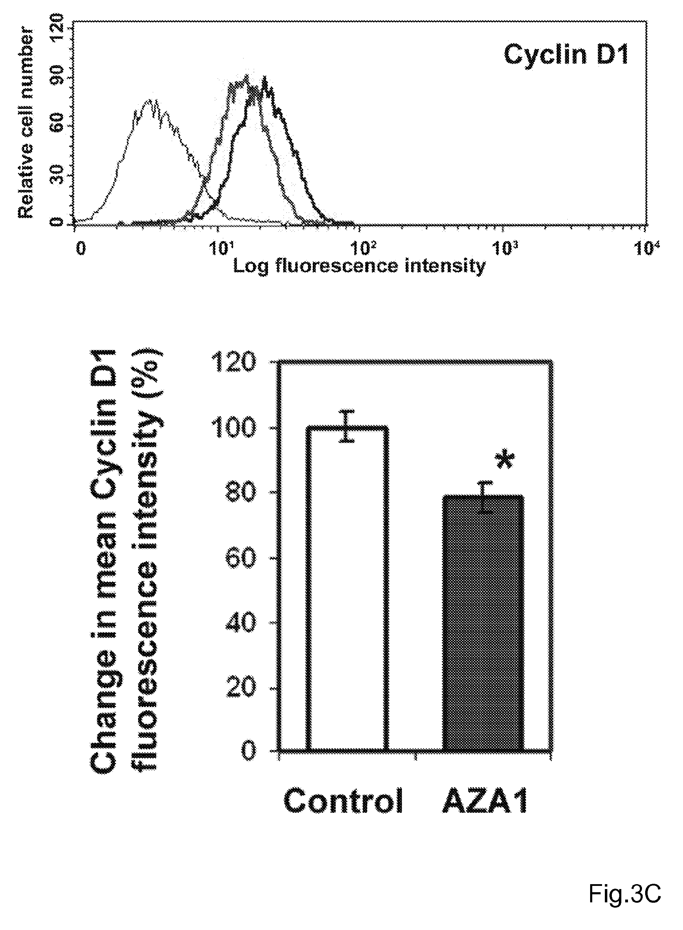

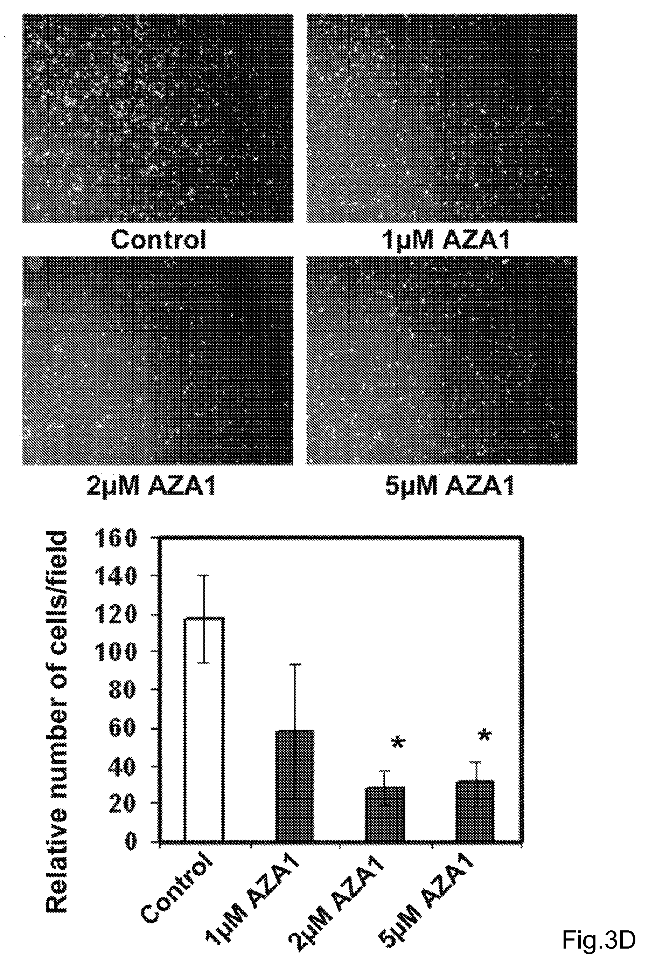

Fig. 3 shows (A) the effect of Rac inhibition by AZA1 on cell proliferation in CRL-2505 prostate cancer cells. Relative cell densities of CRL-2505 cancer cells up to 72 h following treatment with 1, 2, 5, 10, and 25 µM compound AZA1 were measured using the WST-1 cell proliferation assay. Treatment with compound AZA1 suppresses CRL-2505 prostate cancer cell proliferation in a dose-dependent manner. Means of three independent experiments are shown. (B) Representative flow cytometry histogram showing the population of cells in M1 (Sub-G1), M2 (GO/G1), M3 (S) and M4 (G2/M) phases. CRL-2505 cells were incubated with 1µM and 2µM compound AZA1 for 24 h. Control cells received no treatment. Cellular DNA content was analyzed with flow cytometry after staining. (C) CyclinD1 expression. Representative flow cytometry histogram and quantification of Cyclin D1 fluorescence intensity in CRL-2505 cells treated with compound AZA1 compared to controls. Compound treatment reduced Cyclin D1 levels. *, significantly different vs. control (P<0.05). (D) Rac blockade reduces prostate cancer cell migration. Representative images of migrated macrophages from an in vitro migration assay are shown. Prostate cancer cells were treated with 1, 2, and 5µM compound AZA1 for 24h. Quantification of migrated cancer cells from the in vitro migration assay was determined 24 h after treatment with 1, 2 and 5µM compound AZA1. Data were collected from five individual consecutive fields of view (40x objective) of each of the three replicate Boyden chambers. *, P<0.01, significantly different from control.

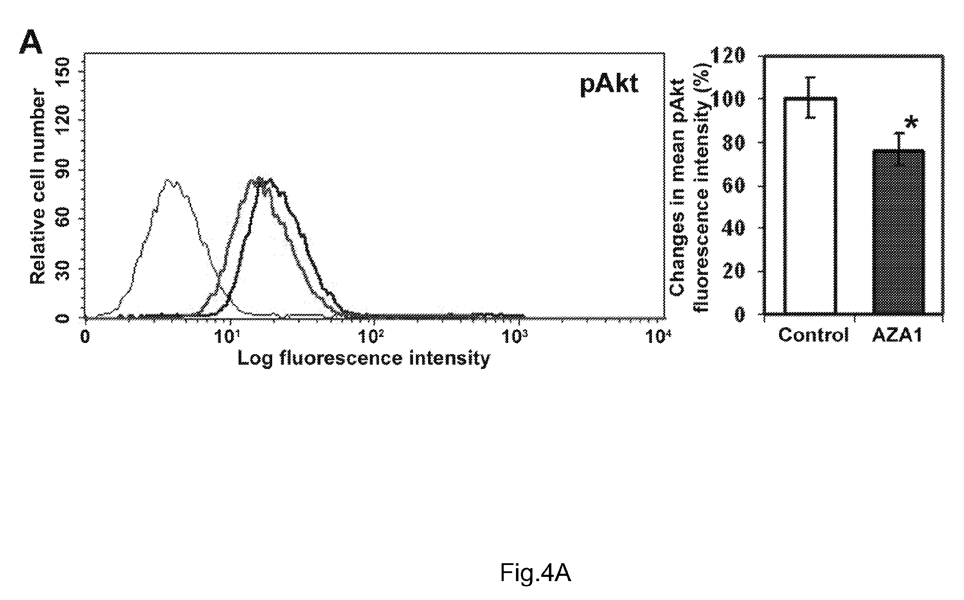

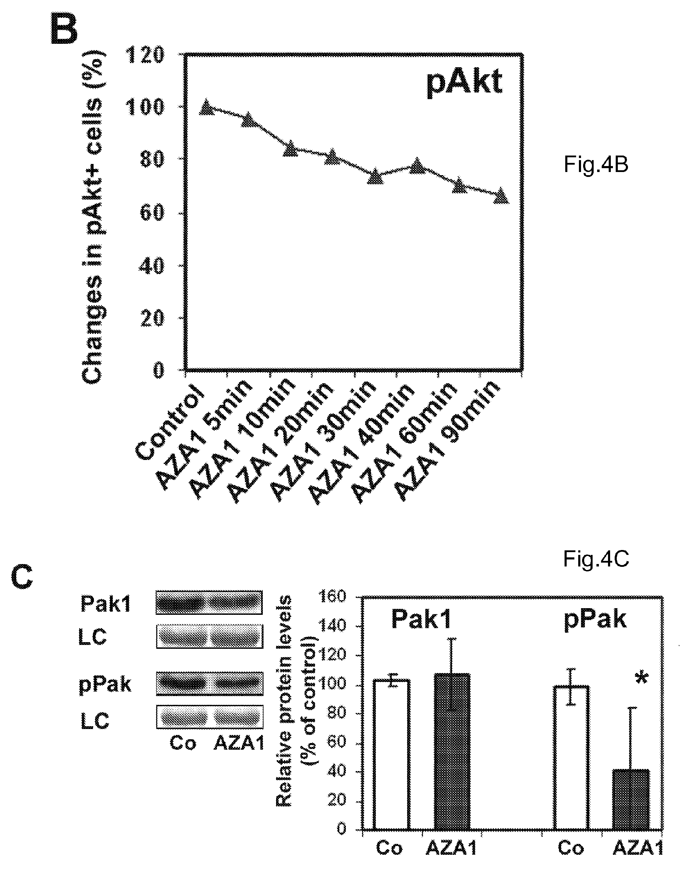

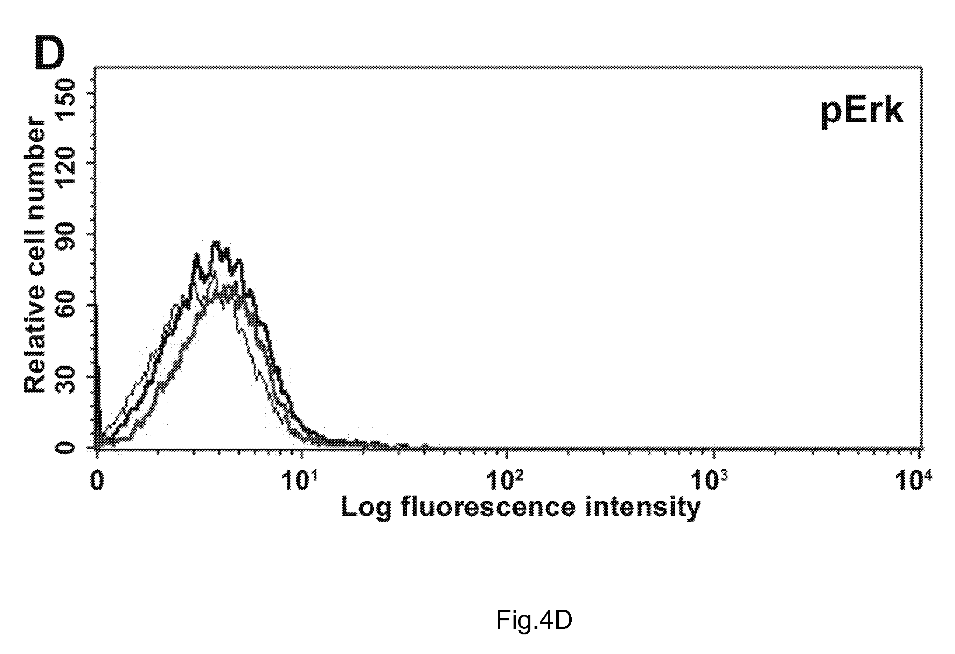

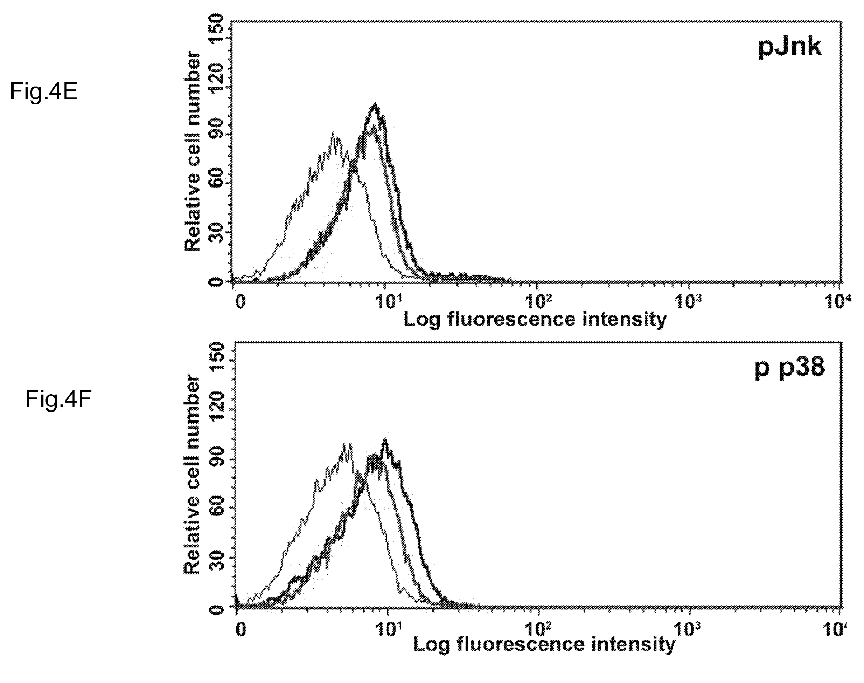

Fig. 4 shows the analysis of Rac-signal transduction effectors in CRL-2505 prostate cancer cells by flow cytometry. (A) Representative histogram and quantification of phospho-Akt (pAkt) fluorescence intensity in prostate cancer cells with and without treatment with compound AZA1. Cells were permeabilized and stained with phycoerythrin conjugated phospho-Akt monoclonal antibody or isotype matched controls (grey histogram). Treatment with 20 µM compound AZA1 for 60 min (red) reduces phosphorylation of Akt in prostate cancer cells as compared to untreated cells (black). Means of 3 independent experiments are shown. (B) Quantification of phospho-Akt fluorescence intensity after treatment with AZA1 using flow cytometry at different time points. Treatment with compound AZA1 reduces phosphorylation of Akt in prostate cancer cells over time (P<0.03). (C) Representative Western blot images and quantification of prostate cancer cells before and after treatment with 2 µM compound AZA1 for 24h stained with Pak1 and phospho-Pak1/2 (pPak) antibodies. Rac blockade reduces Pak1/2 phosphorylation in prostate cancer cells (means of 3 independent experiments) without affecting total protein levels (P<0.03). Co, control . (D) Representative histogram of phospho-ERK (pErk) fluorescence intensity in prostate cancer cells treated with compound AZA1 (red line) compared to untreated cells using flow cytometry. (E) Representative histogram of phospho-JNK (pJNK) fluorescence intensity in prostate cancer cells treated with compound AZA1 (red line) compared to untreated cells using flow cytometry analysis. Phospho-JNK levels are reduced following Rac inhibition (P<0.05). (F) Representative histogram of phospho-p38 (p-p38) fluorescence intensity in prostate cancer cells treated with compound AZA1 (red line) compared to untreated cells using flow cytometry analysis. Phospho-p38 levels are reduced following Rac inhibition. *, P<0.05, significantly different from control.

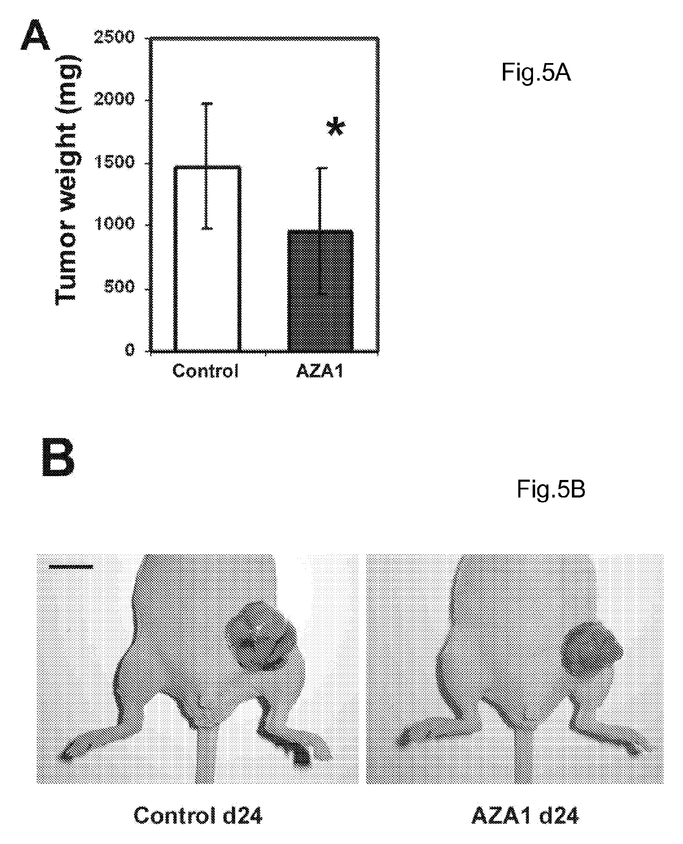

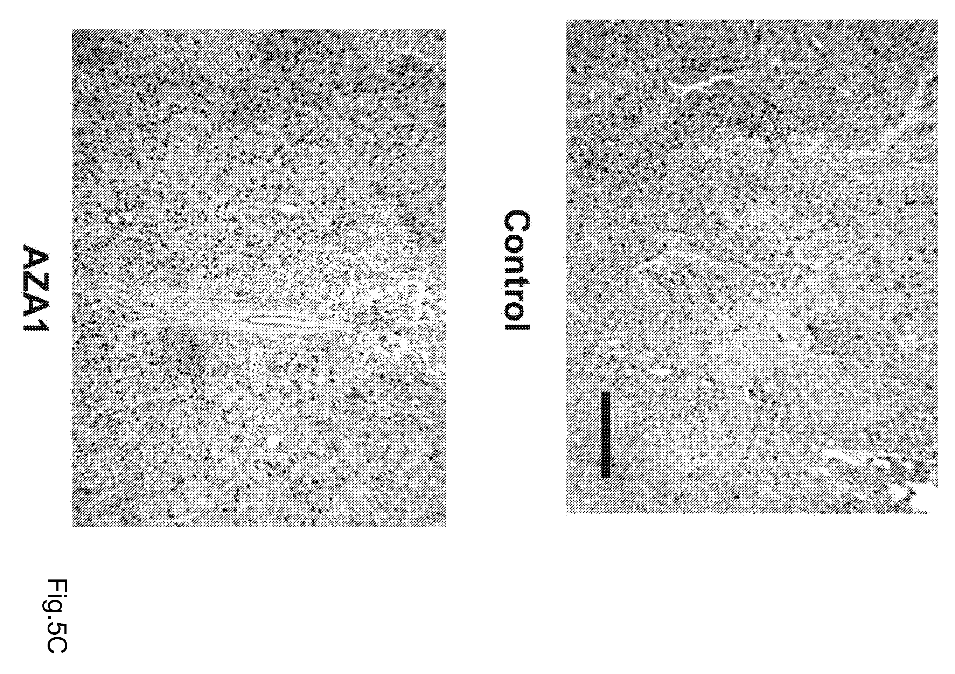

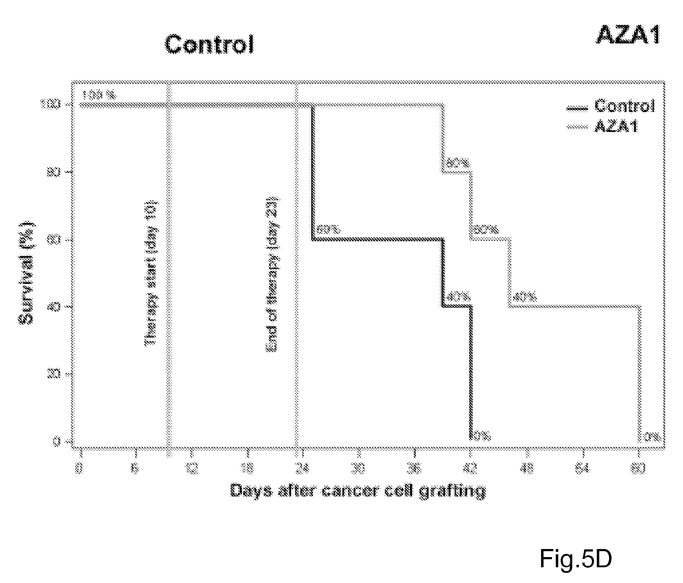

Fig. 5 shows that Rac blockade suppresses tumour growth and prolongs animal survival. (A) Effect of treatment with compound AZA1 on tumour growth. Treatment with AZA1 significantly suppresses tumour growth of human prostate xenografts in mice. Data are shown as mean CRL-2505 prostate cancer xenograft tumour weights on day 24 of control tumors and tumors treated with compound AZA1. *, P<0.03, significantly different from control on day 24. (B) Representative images of human prostate tumour xenografts in a control mouse on day 24 (Control d24) and in a mouse treated with 100 µg/day compound AZA1 on day 24. The tumour size is markedly reduced in mice treated with AZA1 compared to controls. Calibration bar = 1 cm. (C) Representative immunohistochemistry images of tumour tissue sections of prostate tumour xenografts in a control mouse on day 24 (Control d24) and in mice treated with AZA1 (AZA1 d24) stained with Ki-67 antibody. Cellular proliferation is reduced following Rac blockade. Bar = 200 µm. (D) Effect of compound AZA1 treatment on animal survival of CRL-2505 prostate cancer bearing mice. Treatment with compound AZA1 significantly prolongs animal survival vs. untreated controls (P<0.05). The overall survival curves after treatment were analyzed by the Kaplan-Meier survival test.

### EXAMPLES

### Example 1:

Synthesis of N*2*-(4-Diethylamino-1-methyl-butyl)-5-methyl-N*4*-(2-methyl-1H-indol-5-yl)-pyrimidine-2,4-diamine, N*2*,N*4*-Bis-(1H-indol-5-yl)-pyrimidine-2,4-diamine, N*2*,N*4*-Bis-(2-methyl-1H-indol-5-yl)-pyrimidine-2,4-diamine and N*2*-(1H-Indol-6-yl)-N*4*-(1H-indol-5-ylmethyl)-pyrimidine-2,4-diamine are hereinafter also referred to as compounds "10", "41", "46" ("AZA1") and "55" respectively, can be produced by well available methods and syntheses. Compounds 10, 41, 46 (AZA1) and 55 used in the present examples have been produced according to the following method (in the formulae, H atoms are not depicted):

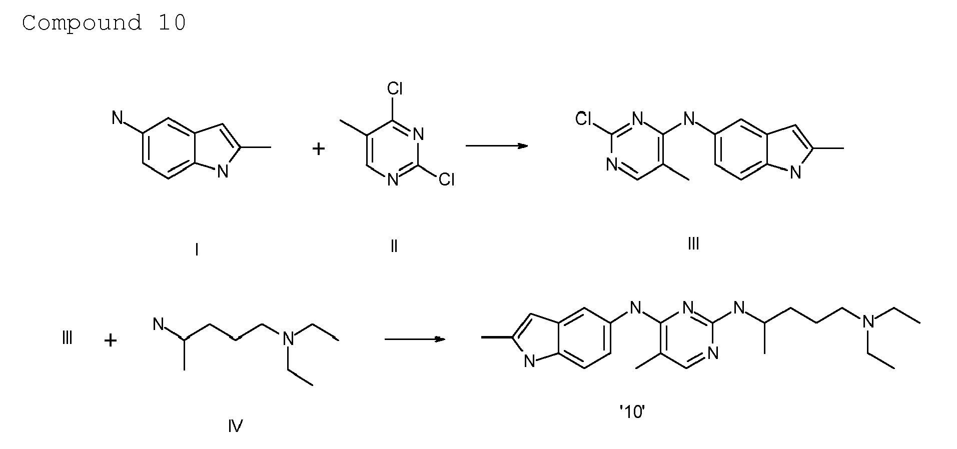

An equimolar amount of compounds I and II were reacted in MeCN using Et₃N as a base. Compound III was liberated by column chromatography from its unwanted isomer and reacted with amine IV, neat, under microwave conditions. Purification by column chromatography gave pure 10.

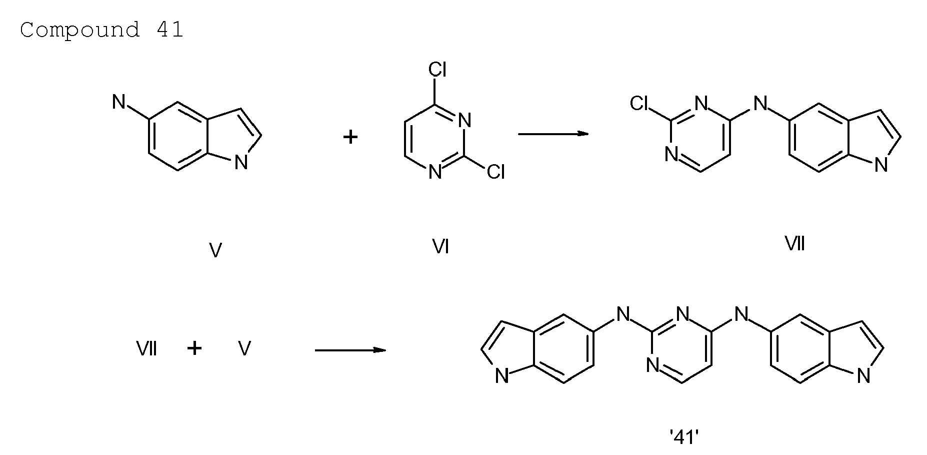

An equimolar amount of compounds V and VI were reacted in MeCN using Et₃N as a base. Compound VII was liberated by column chromatography from its unwanted isomer and reacted with amine V in EtOH with N,N-diisopropylethylamine as the base. Purification by column chromatography gave pure 41.

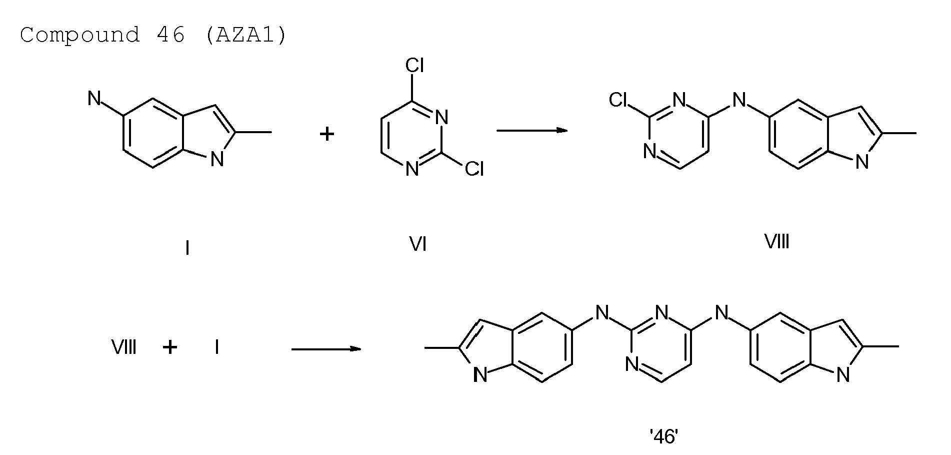

An equimolar amount of compounds I and VI were reacted in MeCN using Et₃N as a base. Compound VIII was liberated by column chromatography from its unwanted isomer and reacted with amine I in EtOH with N,N-diisopropylethylamine as the base. Purification by column chromatography gave pure 46 (A2A1).

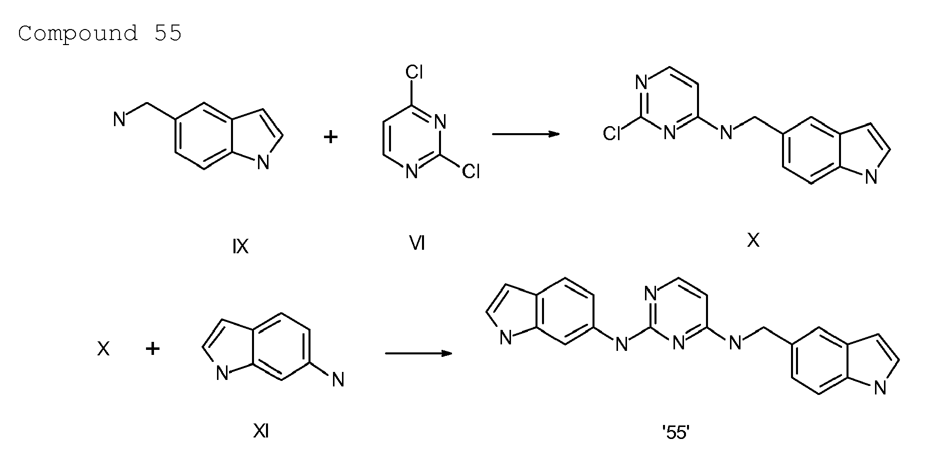

An equimolar amount of compounds IX and VI were reacted in MeCN using Et₃N as a base. Compound X was liberated by column chromatography from its unwanted isomer and reacted with amine XI in EtOH with N,N-diisopropylethylamine as the base. Purification by column chromatography gave pure 55.

### Example 2:

### Screening process for Rac1 inhibitor compounds:

Compounds 1 to 200 as depicted in Figs 1a, 1b and 1c were selected based on their practicality for synthesis, toxicity and solubility. Compounds 1, 2, 3, 4, 6, 10, 13, 14, 17, 22, 25, 26, 31, 40, 41, 44, 46 (AZA1), 55, 56, 72 and 120 according to Fig. 1 were further investigated for solubility and toxicity.

Compounds in powder form were dissolved in 100% DMSO to obtain 100 mM stock solutions of each of the compounds. Solubility of the compounds was then tested by preparing solutions in 20-30% DMSO in water. A compound was selected to have appropriate solubility if 1mM of the compound is soluble in 20-30% DMSO. Compounds which were non-soluble in 30% DMSO were regarded as non-soluble. NSC23766 was always used as a reference compound.

Compounds 2, 4, 6, 10, 13, 14, 22, 25, 26, 40, 41, 46 (AZA1), 55, 56, 72 and 120 were selected due to appropriate solubility.

### Example 3:

### Biochemical analysis of AZA1 and preclinical trials for AZA1 in a prostate cancer animal model

### Material and Methods

### Cell lines.

Human 22Rv1 prostate cancer cells (CRL-2505) were obtained from American Type Culture Collection (ATCC; Manassas, VA) and cultured in Dulbecco's modified Eagle's medium (DMEM, PAA Laboratories, Pasching, Austria) supplemented with 10% fetal calf serum (FCS; PAA Laboratories), 0.1M nonessential amino acids, 100 U/ml penicillin and 100 µg/ml streptomycin.

### Compound generation.

Based on the structure-function information on Rac1-GEF interaction and the identified compound NSC23766 as an inhibitor of Rac1 activation (Gao et al., 2004) and a study utilizing a virtual screening strategy using the ZINC database to discover new classes of Rac1 inhibitors (Ferri et al., J. Med. Chem. 52 (2009), 4087-4090), 21 new chemically diverse potential Rac-inhibiting compounds were generated, which were subsequently tested in vitro by solubility examination, Rac activation assays and mitochondrial toxicity assays (WST-1) as outlined below. Based on the latter experiments four potential chemical compounds were identified, which were then tested by using in vivo human prostate cancer xenograft models. The formulas of these compunds were:
AZA1 ("Compound 46"): N*2*,N*4*-Bis-(2-methyl-1H-indol-5-yl)-pyrimidine-2,4-diamine;
AZA2: ("Compound 10") N*2*-(4-Diethylamino-1-methyl-butyl)-5-methyl-N*4*-(2-methyl-1H-indol-5-yl)-pyrimidine-2,4-diamine;
AZA3: ("Compound 41") N*2*,N*4*-Bis-(1H-indol-5-yl)-pyrimidine-2,4-diamine; and
AZA4 ("Compound 55"): N*2*-(1H-Indol-6-yl)-N*4*-(1H-indol-5-ylmethyl)-pyrimidine-2,4-diamine.

The latter in vivo experiments then identified compound AZA1 as the most potent agent with regard to solubility, lack of toxicity and tumor-suppressive effects.

### Rac 1, 2, 3 activation assay.

Prostate cancer cells were seeded in 6-well plates and starved for 20 h. Cells were incubated with Rac1 small molecule inhibitor AZA1 for 4 h and then stimulated with 50 ng/ml epidermal growth factor (EGF) (R&D systems) for 1 min 30 sec and Rac activation was then measured with the G-LISA method (Rac 1, 2, 3 activation assay, colorimetric format, Cytoskeleton) according to the manufacturer's protocol.

### Cell proliferation assay.

Human CRL-2505 cells were seeded in 96-well plates at a density of 1x10⁴ cells/well in culture medium. Cells were incubated with 1, 2, 5, 10, 25 µM compound AZA1. Cell proliferation was determined 24, 48 or 72 h after treatment using the WST-1 reagent (Roche Diagnostics, Indianapolis, IN) according to the manufacturer's protocol (Zins et al., Cancer Res. 67 (2007), 1038-1045). Each experiment was repeated three times.

### Migration assay.

Prostate cancer cells (5x10⁴ in 1 ml DMEM with 10% FCS) were added to the top of each Boyden migration chamber (8-µm, 12-well plate format; BD Biosciences, Palo Alto, CA). Cells were incubated with 1, 2, and 5 µM of compound AZA1. After 24 h, medium was removed and membranes were washed twice with PBS. Cells from the upper side of the membrane were removed with cotton swabs. The membranes were excised using a scalpel, inverted and transferred to a PBS filled tissue culture well. Membranes were then fixed in methanol for 2 min at -20°C. After washing in PBS, membranes were stained with 1 µg/ml 4'-6-Diamidino-2-phenylindole (DAPI) in PBS for 10 min at RT and washed again in PBS. Membranes were then embedded in Cityfluor (Cityfluor, Leicester, UK) on glass slides under coverslips. Representative sectors of migrated prostate cancer cells were counted under a fluorescence microscope. Each experiment was performed in triplicate.

### FACS analysis.

Tumour cells were seeded in 10 cm plates and allowed to adhere before treatment with 20 µM compound AZA1 for 4 h before trypsinization and washing with PBS.

One portion of the cells were then fixed with 70% ethanol for 1h at 4°C, washed with PBS and stained with propidium iodide (PI) puffer with 50 µg/ml DNase-free RNaseA. Cells in different cell cycle stages were determined. The rest of the cells were fixed with Cytofix fixation buffer (BD) for 30 min at 37°C, washed and then permeabilized with Perm buffer III (BD) and stained with Cyclin D1 (anti-human Cyclin D1 antibody set), phospho-Akt (anti-phospho-Akt pT308), phospho-ERK (anti-phospho-ERK1/2 pT202/pY204), phospho-JNK (anti-phospho-JNK pT183/pY185) and phospho-p38 antibodies (anti-phospho-p38 MAPK pT180/pY182) (the latter antibodies were purchased from BD Biosciences). 104 events were analyzed on a FACScan flow cytometer (BD Biosciences) with an argon laser tuned to 488 nm.

### Western blotting.

Cell lysates were prepared (Aharinejad et al., Cancer Res. 62 (2002), 5317-5324 and Cancer Res. 64 (2004), 5378-5384) and 50 µg/lane separated by 9% SDS-PAGE prior to electrophoretic transfer onto Hybond C super (Amersham Pharmacia Biotech, Buckinghamshire, UK). The blots were probed with antibodies against PAK1 and phospho-Pak1 (pS144)/Pak2 (pS141) (Cell Signaling Technology, Danvers, MA) before incubation with horseradish peroxidase-conjugated secondary antibodies (Amersham Pharmacia Biotech). Proteins were immunodetected by chemiluminescence (Supersignal-West- Femto, Pierce, Rockford, IL) and quantified by Image Quant 5.0 software (Molecular Dynamics, Sunnyvale, CA).

### Tumour models.

The experiments performed in this study were approved by the Institutional Animal Care and Use Committee at the Vienna Medical University. Pathogen-free, male, 5 week-old athymic nu/nu (nude) mice (Charles River, Sulzfeld, Germany) were weighed, coded and divided into experimental groups of (n = 10) at random. Mice were anesthetized (ketamine hydrochloride/xylazine at 55/7.5 mg/kg i.p.) and 15x10⁶ CRL-2505 cells/100 µl PBS were injected s.c. into the left flank (Aharinejad et al., 2004). In a preliminary experiment mice received i.p. injections with 100 µg compound AZA1, AZA2, AZA3, or AZA4 in 100 µl 30% DMSO six days after cell injection for two weeks, control animals received 100 µl 30% DMSO/day (n = 5 for each group). In the main experiment, mice received i.p. injections with 100 µg compound AZA1 in 100 µl 30% DMSO six days after cell injection for two weeks, control animals received 100 µl 30% DMSO/day (n = 10 for each group). Tumour volumes were calculated as length x width²/2. All animals were sacrificed on day 20.

### Analysis of the effects of compound AZA1 in vivo.

On day 24, tumors were isolated, weighed and the animals were sacrificed. One portion of the tissue was processed for paraffin embedding. Paraffin-embedded serial sections were rehydrated in graded alcohols and antigen retrieval performed in a microwave in 0.1 M sodium citrate (pH 6.5). Following incubation in 5% H₂O₂ to block endogenous peroxidase activity, antigens were detected with Ki-67 antibody (tumour proliferation assay; Dako, Glostrup, Denmark) to evaluate the density of proliferating cells (Aharinejad et al., 2002 and 2004). Primary antibodies were detected by sequential incubation with appropriate biotinylated secondary antibodies (Vector Laboratories, Burlingame, CA) and peroxidase conjugated streptavidin (Dako), developed with 3, 3'-diaminobenzidine (Vector Laboratories), counter-stained with haemalaun, dehydrated and mounted in DPX (Merck, Darmstadt, Germany) and digitalized images were generated.

### Analysis of the effects of compound AZA1 on survival.

The survival study was set for three months. Mice were treated with compound AZA1 (n = 10) or 30% DMSO (n = 10) and were euthanized when moribund.

### Statistical analysis.

Data were tested for normality using the Shapiro-Wilk test. Groups were compared by nonparametric analysis of variance (ANO-VA, Wilcoxon rank-sum test, Kruskal-Wallis test). All statistical tests were two-sided. The overall survival curves after treatment were analyzed by the Kaplan-Meier survival test. Statistical tests were performed with the use of SAS software (version 9.1.3) and Enterprise Guide (version 4.1, SAS Institute Inc., Cary, NC). Data are expressed as means ± SD. P values of < 0.05 were considered to indicate statistical significance.

### Results

### Treatment with the small molecule inhibitor AZA1 inhibits Rac1 activity in human CRL-2505 prostate cancer cells.

Rac activation in CRL-2505 prostate cell lysates following stimulation with epidermal growth factor (EGF) was examined first. The activation of EGF receptor (EGF-R) plays a key role in the promotion of proliferation and invasion in prostate cancer, and thus the use of EGF for activation of the EGF-R signaling pathways constitutes a pathologically relevant factor for analysis of prostate cancer growth in the CRL-2505 model. The activity of Rac was threefold upregulated after stimulation of prostate cancer cells with EGF as compared to untreated cells. Next, the effect of the 21 newly designed small molecule Racinhibitors on Rac activity following EGF treatment was tested. The most effective Rac inhibitor was selected for further analyses (AZA1). Treatment of CRL-2505 human prostate cancer cells with 5, 10 and 20 µM AZA1, reduced Rac activity significantly and this reduction was dose-dependent (Fig. 2).

### Blockade of Rac1 activity by AZA1 inhibits cell proliferation in human CRL-2505 prostate cancer cells.

The decrease in cell viability could result from reduced cell proliferation and/or apoptosis in the target cells. To distinguish these effects, it was tested whether compound AZA1 influences cancer cell proliferation and apoptosis. Human CRL-2505 prostate cancer cells were treated with different concentrations of compound AZA1 and the increase in mass of cellular protein was determined for up to 72 hours. CRL-2505 cell proliferation was significantly reduced after 72 hours of incubation with 1, 2, 5, 10 and 25 µM of compound compared to untreated cells. The effect of compound AZA1 on cell proliferation was dose-dependent (Figure 3A). These data indicate that treatment with compound AZA1 reduces prostate cancer cell proliferation.

Next, cell cycle distribution following AZA1 treatment was analyzed. Cells were gated into sub G1 (M1), G0/G1 (M2), S (M3) and G2/M (M4) phases. The sub G1 (M1) population was used to estimate apoptosis. Figure 3B shows a representative set of data from untreated and AZA1-treated CRL-2505 cells. After 24 h, CRL-2505 cells treated with 1 µM and 2 µM compound AZA1 showed an increase in sub G1 events (M1) from 1.47% to 8.08% and 9.26%, respectively, and a decrease in G2/M (M4) phases from 32.25% to 28.44% in cells treated with 2 µM AZA1 (Figure 3B), suggesting inhibition of cellular proliferation and an increase in the number of apoptotic cells.

Then the effect of Rac1 inhibition on the cell cycle regulatory protein Cyclin D1 was examined. In cell lysates treated with Rac compound, Cyclin D1 fluorescence intensity decreased significantly by 22% compared to untreated controls as shown in Figure 3C. Next, flow cytometry was used to assess apoptosis in response to Rac1 inhibition. CRL-2505 cells showed no significant signs of apoptosis 3 h after AZA1 treatment, as indicated by staining with Annexin V-FITC and PI. Taken together, these results support a role of AZA1 in blocking Rac1-dependent cell cycle events in CRL-2505 prostate cancer cells without a major impact on the induction of apoptosis.

### Compound AZA1 reduces cancer cell migration.

Since cancer cell migration is frequently observed in growing tumors, the effect of compound AZA1 on cancer cell migration was investigated in an in vitro migration assay. Treatment of cells with 2 µM and 5 µM compound for 20 h significantly reduced migration compared to untreated controls (Fig. 3D). In contrast, treatment with 1 µM compound failed to reduce cancer cell migration. These data show a role for AZA1 compound in blocking Rac1dependent cancer cell migration.

### Inhibition of Rac leads to downregulation of the Akt and Pak signaling pathway.

Rac inhibition by AZA1 led to a significant inhibition of phospho-Akt levels (a reduction of 24%) that correlated with Akt activity (Figure 4A). pAkt was also determined at different time points and decreased significantly at 5 min to 90 min after treatment with compound AZA1 (Fig 4B).

Then the role of Rac inhibition on Pak phosphorylation in cancer cells was investigated. Pak1 expression was not changed in cells compared to controls following compound treatment, whereas Pak1/2 phosphorylation at serine 144/141 was significantly reduced, indicating that Rac inhibition also blocks the Pak1 signaling pathway (Fig. 4C). In contrast, the activity of the potential Rac1 effector ERK was not affected by Rac1 inhibition (Figure 4D). AZA1 treatment, however, caused a significant decrease of phosphorylation of two other MAPK pathways, JNK and p38 (Figure 4E and 4F), indicating that activation of these MAPK pathways is regulated by Rac1 in CRL-2505 prostate cancer cells. These results show that Rac1 targeting strategy can modulate the Pak/Akt signaling pathway and MAP kinase pathways to affect prostate cancer cell survival and proliferative response.

### Rac1 blockade suppresses primary prostate cancer growth and prolongs animal survival.

To analyze whether tumour cell-derived Rac plays a critical role in tumour growth, mice bearing human prostate cancer xenografts were treated with Rac inhibitor AZA1 or vehicle (30% DMSO). To assess treatment modalities in vivo, Rac inhibitor stability in vitro were initially assessed and cycled treatment daily for two weeks to guarantee continuous reduction of tumour cell-derived Rac. At the beginning of treatment on day 6, mice developed human tumors of comparable size. At day 24 the mean tumour weight was markedly reduced in mice treated with Rac inhibitor (956 mg ± 505 mg) compared to control mice (1470 mg ± 497 mg) (Figs. 5A, B). In addition, cell proliferation, assessed by staining with Ki-67 antibody, was reduced following treatment with compound AZA1 (Fig. 5C), suggesting an anti-proliferative effect of Rac inhibition by the compound AZA1.

To analyze the long-term effect of Rac blockade on prostate cancer growth, the effect on animal survival was determined. In mice bearing prostate cancer xenografts, the median time to death in the control group was 39 ± 15 days, and all mice died between 25 and 42 days after tumour cell grafting. However, survival was significantly increased in mice following Rac inhibitor treatment vs. control mice, and their median time to death was 46 ± 4 days. At day 42 (at which time the last animal in the control groups died), 60% of animals treated with the Rac inhibitor were still alive (Fig. 5D). Rac compound treated mice exhibited improved survival, indicating a negative role for activated Rac on survival of prostate cancer bearing animals.

### Discussion

The present example shows the effectiveness of the preferred compound according to the present invention in a representative tumour model. The prostate cancer model chosen for the present example was used because it is an established tumour model and a model which can also serve as proof of principle for other malignant diseases.

On the other hand, prostate cancer is also a highly relevant example for a malignant disease and treatment of prostate cancer patients a preferred embodiment of the present invention. Prostate cancer is one of the most common malignant tumour types and the second leading cause of cancer death in men in North America and Western Europe. Therefore, novel treatment modalities in prostate cancer patients are urgently needed. However, a current challenge is to design novel therapeutic strategies that specifically target prostate cancer cells.

In this context, increased Rho proteins, including Rac1, may be involved in the proliferation of cancer cells and/or response to apoptotic cell death signals. Furthermore, expression of a constitutively active Rac1 mutation Val-12 Rac1 (Rac1 with valine at residue 12) was found to be an important component of Ras-induced transformation, displaying all the hallmarks of malignant transformation. However, unlike Ras, a constitutively active mutation of Rac has not been found in human cancer and the specific mechanisms by which Rac1 influences transformation and tumour progression are not fully understood. Importantly, Rac1 overexpression occurs in many tumour types including cancers of the breast, lung and colon and Rac protein expression was also reported to be significantly increased in prostate carcinomas. In additon, Rac1 is involved in cell adhesion and migration and Rac1 activation is associated with increased tumour invasiveness. This suggests that upregulation or overexpression, rather than an activating mutation of Rac is associated with Rac tumorigenic properties, findings that also have important therapeutic implications.

Consequently, it is now clear that Rac1 is involved in multiple stages of the tumorigenic process, suggesting that Rac1 and its effectors are useful anticancer targets. In line with this, previous studies showed that inhibition of Rac by a small molecule inhibitor NSC23766 inhibits PC-3 prostate cancer cell proliferation, anchorage independent growth, and invasion of the Rac-hyperactive human PC-3 cancer cell line in vitro and inhibited proliferation of chronic myelogenous leukemia (CML) in cells in vitro and in vivo.

However, as the pathways regulated by Rac are vast, inhibitors of Rac signaling have a risk of being highly toxic, practically making these inhibitors unusable in cancer therapy. In order to develop better Rac inactivators, small chemical compounds based on the principal structure of compounds interfering with GEF binding to Rac have been designed and screened these newly designed compounds for improved down-regulation of endogenous Rac1 activity as compared to the known inhibitor NSC23766. Screening of the different compounds identified a novel Rac inactivating compound, named AZA1 (N2,N4-Bis-(2-methyl-1H-indol-5-yl)-pyrimidine-2,4-diamine) showing improved Rac inhibiting activity when compared to NSC 23766, in CRL-2505 prostate cancer cells. AZA1 blocked physiological Rac1 activity in the human prostate cancer cell line CRL-2505, as well as cancer cell proliferation and migration. To examine the efficacy of the AZA1 compound in vivo, it was tested in immune-deficient mice xenografted with human CRL-2505 prostate cancer cells. It was shown that 2 weeks of systemic AZA1 treatment selectively suppressed the growth of the human prostate cancer xenografts and significantly improved animal survival.

Several lines of evidence support an important role of Rac1 signaling for cellular transformation via modulation of antiapoptotic and cell cycle mechanisms. The PI3K/Akt signaling pathway is known for its role in mediating cell survival, as well as cell cycle progression and neoplastic transformation and appears to be critical to prostate cancer cell survival and proliferation. The present data show a significant reduction in phosphorylation and hence activation of Akt following Rac-inhibition by AZA1. Activated Akt translocates to the cytoplasm and nucleus and activates downstream targets involved in survival, proliferation, cell cycle progression, growth and cell migration. The results presented here demonstrate an inhibition of cancer cell proliferation in vitro and staining with the cell proliferation marker Ki-67 is reduced in cancer tissue in vivo following Rac-inhibition. Furthermore, reduced expression of Cyclin D1 fits to these findings, since data suggest that Rac1 can influence transformation through regulation of Cyclin D1, a cell cycle protein that is frequently overexpressed in cancer including prostate carcinoma xenografts and metastases. Cyclin D1 is known to regulate the cell cycle by stimulating phosphorylation of retinoblastoma protein (Rb) protein, which subsequently triggers transcription of various genes required for G1 progression and overexpression of cyclin D1 has been reported to increase tumorigenicity of the LNCaP prostate cancer cell line. Of interest, Cyclin D1 expression may be regulated in prostate cancer as a consequence of Akt-mediated activation, which has been shown to lead to overexpression of several key proteins include Cyclin D1, and this hypothesis is further supported by the present data.

Akt can also be activated by the Rac1 effector, p21 activating kinase 1 (Pak1), which was shown to activate Akt by direct interaction. Pak was among the first described Rac1 effector proteins and it binds Rac1 in a GTP-dependent manner, potently stimulating Pak kinase activity. Rac1 signals through Pak to activate JNK. Although Rac1 activation was found to promote cell survival by activation of Akt, Erk and NF-kB, it can also induce apoptosis by stimulation of p38 and JNK. However, in the CRL-2505 prostate cancer cells analyzed here, phosphorylation of p38 and JNK was reduced following Rac-inhibition associated with decreased cell proliferation and no major signs of increased apoptosis. Thus, as p38 and JNK are known to be antiproliferative and proapoptotic, the observed decreased activation of p38 and JNK following Rac-inhibition by AZA1 suggest that these pathways play no major tumour promoting role in this context and that other Rac effector pathways mediate its proliferative effect in CRL-2505 prostate cancer. Another study reported that activation of JNK and p38 was severely decreased in Rac1 and Rac2-deficient splenocytes expressing p210 Bcr-Abl, suggesting a possible involvement of these pathways in disease progression, which would be in line with the present findings. Furthermore, in human prostate cancer cells retaining a functional p53 protein (LNCaP and CRL-2505) cellular senescence but not apoptosis is the dominant form of death following radiation therapy, which further supports the present findings.

In addition to its effect on proliferation, Rac inhibition also reduced prostate cancer cell migration. A recent report showing that Pak1 signaling is critical to medulloblastoma cell migration suggests that reduced Pak1 activity in the present prostate cancer model may account for this effect on cell migration. Because Pak1 can also regulate cell transformation and survival, and because of its increased frequency of phosphorylation observed in poor outcome tumors, the Pak signaling pathway could therefore constitute a major effector pathway of Rac1 in prostate cancer.

In contrast to JNK and p38, ERK activity remained unchanged in cancer cells following AZA1 treatment. Comparable to the present results, downregulation of Pak1 in medulloblastoma cells did not alter platelet-derived growth factor mediated activation of ERK and a study in osteoclast cells similarly showed that Pak1 does not modulate Raf-mediated MEK activation by macrophage colony stimulating factor. Moreover, although the MEK/ERK MAPK pathway is known for its role in cell proliferation, there is little evidence that MAPK activity is increased in prostate cancer and interactions between Rac1/Pak1 and MEK/ERK is likely cell type-dependent. In addition, ERK activity was not affected by Rac1 inhibition in lymphoma cells further suggesting that Rac1 targeting strategy exerts its effect via modulation of the Pak1-Akt signaling pathway, but not the MAP kinases, to affect prostate cancer cell proliferation.

In summary, the present invention describes a second generation small molecule Rac-inhibiting substance. Evidence is provided that Rac activity is downregulated by AZA1 in CRL-2505 prostate cancer cells and that Rac suppression impairs cell proliferation and reduces activation of Akt and Pak signaling pathways. Furthermore, it is shown that Rac inhibition in vivo reduces tumour growth and prolongs survival in a prostate cancer xenograft mouse model. Together, these observations identify AZA1 as a Rac-targeting small molecule inhibitor for pharmacologic intervention in the treatment of prostate cancer. On the other hand, especially the results obtained and the mechanisms analysed in the present example clearly show that the compounds according to the present invention can be efficiently used in the treatment of other types of cancer. Accordingly, the use of the compounds according to the present invention in the treatment of all malignant diseases where these mechanisms are relevant is specifically preferred.

## Claims

1. : Compound selected from the group N*2*,N*4*-Bis-(2-methyl-1H-indol-5-yl)-pyrimidine-2,4-diamine, N*2*-(4-Diethylamino-1-methyl-butyl)-5-methyl-N*4*-(2-methyl-1H-indol-5-yl)-pyrimidine-2,4-diamine, N*2*,N*4*-Bis-(1H-indol-5-yl)-pyrimidine-2,4-diamine and N*2*-(1H-Indol-6-yl)-N*4*-(1H-indol-5-ylmethyl)-pyrimidine-2,4-diamine, preferably N*2*,N*4*-Bis-(2-methyl-1H-indol-5-yl)-pyrimidine-2,4-diamine,
and any of its stereoisomeric forms or a mixture thereof;
or a salt or solvate of a compound of these compounds, especially for use in the treatment of malignant diseases.

2. : N*2*,N*4*-Bis-(2-methyl-1H-indol-5-yl)-pyrimidine-2,4-diamine or a salt or solvate thereof for use in the treatment of malignant diseases.

3. : A pharmaceutical preparation containing a compound according to claim 1 or 2 and a pharmaceutically acceptable carrier.

4. : A pharmaceutical preparation containing a compound according to claim 1 or 2 and a pharmaceutically acceptable carrier for administration by injection.

5. : Compound according to claim 1 or 2 **characterised in that** said malignant disease is a carcinoma, preferably breast, colon, rectum, esophagus, stomach, liver, lung, renal, thyroid, ovary, uterus, vagina, pancreas, bladder, prostate, skin, head and neck squamous cell carcinoma (HNSCC), and oral squamous cell carcinoma (OSCC), a hematopoietic malignancy of lymphoid lineage, preferably anaplastic large cell lymphomas (ALCLs), p210-BCR-ABL-dependent leukemia and hairy cell leukemia), a hematopoietic malignancy of myeloid lineage, preferably chronic myelogenous leukemias (CML), a malignancy of mesenchymal origin, preferably rhabdomyosarcoma, or a malignant tumour of neuronal origin, especially glioblastoma.

6. : Compound according to claim 1 or 2 **characterised in that** said malignant disease is prostate cancer.

7. : Use of a compound as defined in claim 1 or 2 for the preparation of a pharmaceutical composition for the treatment of a malignant disease.

8. : N*2*,N*4*-Bis-(2-methyl-1H-indol-5-yl)-pyrimidine-2,4-diamine, N*2*-(4-Diethylamino-1-methyl-butyl)-5-methyl-N*4*-(2-methyl-1H-indol-5-yl)-pyrimidine-2,4-diamine, N*2*,N*4*-Bis-(1H-indol-5-yl)-pyrimidine-2,4-diamine or N*2*-(1H-Indol-6-yl)-N*4*-(1H-indol-5-ylmethyl)-pyrimidine-2,4-diamine and any of its stereoisomeric forms or a mixture thereof;
or a salt or solvate of these compounds.
